# EUROPEAN PATENT APPLICATION

(11) **EP 2 382 978 A2**
(43) Date of publication of application: **02.11.2011**
(21) Application number: 11175008.9
(22) Date of filing: 09.06.2006
(51) Int. Cl.: A61K 31/683, A23L 1/30, A61K 9/00, A23J 7/00, A61K 45/06, A61P 17/06, A61P 37/00

(54) **Modulation of the immune system by inositol phospholipids**

(62) Divisional of application: 06747568.1
(71) Applicant: Erasmus University Medical Center Rotterdam, 3015 GD Rotterdam (NL)
(72) Inventor: Nieuwenhuis, Edward Eelco Salomon, 3015 GE Rotterdam (NL); Bruining, Gerbrand Jan, 3015 GE Rotterdam (NL); Samsom, Janneke Nicoline, 3015 GE Rotterdam (NL)
(74) Representative: Jansen, Cornelis Marinus

(57) **Abstract**

The invention relates to the field of immunology. More specifically, it relates to methods and compositions to suppress the immune system, among other by interference with antigen-presenting molecules and T-cell activation. Provided is a composition for the prevention or treatment of a condition wherein suppression of T cell activation is desirable, comprising as an active ingredient an inositol phospholipid or a pharmaceutically acceptable salt thereof wherein the inositol phispholipid is a diacylglycerol phosphotidyl inositol. Said condition can be an auto-immune disease, for example psoriasis.

## Description

The invention relates to the field of immunology. More specifically, it relates to methods and compositions to suppress the immune system, in particular to compositions comprising phospholipids for the prevention or the treatment of diseases that are related to or caused by antigen-presenting molecules and T-cell activation, such as asthma and colitis.

The immune system defends the body from attack by invaders recognized as foreign. At the heart of the system is the ability to recognize and respond to substances called antigens, whether they are infectious agents or part of the body (self antigens). The immune system produces cytokines and other humoral factors to protect the host when threatened by inflammatory agents, microbial invasion, or injury. In most cases this complex defence network successfully restores normal homeostasis, but at other times the immunological mediators may actually prove deleterious to the host. Some examples of immune disease and immune system-mediated injury include anaphylactic shock, autoimmune disease, and immune complex disorders.

Most immune system cells are white blood cells, of which there are many types. Lymphocytes are one type of white blood cell, and two major classes of lymphocytes are T cells and B cells. T cells are critical immune system cells that help to destroy infected cells and coordinate the overall immune response. The T cell bears the T-cell receptor (TCR) on its surface. This receptor interacts with antigen-presenting molecules, such as MHC (major histocompatibility complex) molecules.

MHC molecules are present on the surfaces of most other cells of the body and help T cells recognize peptide antigen fragments. The primary immunological function of MHC molecules is to bind and present antigenic peptides on the surfaces of cells for recognition (binding) by the antigen-specific TCR of lymphocytes. Differential cell-type expression of class I and class II MHC molecules correlates with the specialized functions of different types of immune cells. MHC class I expression is widespread on virtually every cell of the body, consistent with the protective function of cytotoxic TC lymphocytes which continuously survey cell surfaces and kill cells harboring metabolically active microorganisms. MHC class II expression is restricted to antigen presenting cells (APCs). Differential structural properties of MHC class I and class II molecules account for their respective roles in activating different populations of T lymphocytes. Cytotoxic T lymphocytes (CTLs) bind antigenic peptides presented by MHC class I molecules. Helper T_{H} lymphocytes bind antigenic peptides presented by MHC class II molecules.

Different antigen degradation and processing pathways produce MHC-peptide complexes where "endogenous" peptides associate with class I molecules and "exogenous" peptides associate with class II molecules. MHC class I molecules bind peptide fragments derived from proteolytically degraded proteins endogenously synthesized by a cell. Small peptides are transported into the endoplasmic reticulum where they associate with nascent MHC class I molecules before being routed through the Golgi apparatus and displayed on the surface for recognition by CTL's. In contrast, MHC class II molecules bind peptide fragments derived from proteolytically degraded proteins internalized by "antigen presenting cells," including macrophages, dendritic cells, and B cells. The resulting peptide fragments are compartmentalized in the endosome where they associate with MHC class II molecules before being routed to the cell surface for recognition by helper T_{H} lymphocytes.

For the T cell to respond to a foreign antigen on the MHC, another molecule on the antigen-presenting cell must send a second signal to the T cell. A corresponding molecule on the surface of the T cells recognizes the second signal. These two secondary molecules of the antigen-presenting cell and the T cell are called co-stimulatory molecules. There are several different sets of co-stimulatory molecules that can participate in the interaction of antigen-presenting cell with a T cell.

Besides the peptide antigen-presenting MHC molecules, there is the CD1 family representing a group of antigen-presenting molecules which can present lipid antigens to T cells. The CD1 family includes group I CD1a, CD1b, and CD1c and group II CD1d proteins. Topologically, they resemble the classical peptide antigen-presenting MHC molecules except that the large, exclusively nonpolar and hydrophobic, antigen-binding groove of CD1 has evolved to present cellular and pathogen-derived lipid antigens to specific T lymphocytes. The hydrophobic groove differs in shape between different family members, conferring unique lipid binding capacities. Most surface CD1 isoforms containing bound hydrophobic ligand can stimulate specific T-cell receptors on the T-cell. Detailed information on CD1 can be found in a review by Brigl and Brenner ("CD1: antigen presentation and T cell function." Annu. Rev. Immunol. 2004; 22:817-90) and references cited therein. CD1d is a particularly interesting family member, as it is the only CD1 molecule that is found in both mice and humans and has the ability to select and stimulate natural killer (NK) T-cells. NKT cells are T cells with an αß TCR. However, they also express some of the cell-surface molecules of NK cells — hence their name. They differ from most T cells in the sharply limited diversity of their αβ TCRₛ, and these respond to glycolipid antigens presented by a cell-surface molecule designated CD1d (rather than to peptide antigens presented by MHC molecules). NKT cells are able to secrete large amounts of either IFN-Y, a major cytokine of Th1 immune responses or IL-4, the major cytokine of Th2 responses. In addition to defending against some infectious agents, NKT cells have been implicated in protecting against autoimmune diseases, graft rejection and tumors. Notably, NKT cells have also been implicated in the pathogenesis of various (autoimmune)-diseases such as asthma, ulcerative colitis and auto-immune hepatitis.

Once the antigen-presenting molecule, be it a peptide- or a lipid-antigen presenting molecule, and the T-cell receptor interact, there are several possible paths that the T cell may take. These include T cell activation, tolerance, or T cell death. One way T cells can respond upon the interaction of the MHC and the T-cell receptor, and the interaction of the co-stimulatory molecules, is to secrete proinflammatory cytokines and chemokines. Exemplary cytokines include TNF-alpha, interferon-gamma and other interleukins.

The inflammatory response thus plays an important role in limiting and controlling pathogenic infections. Proinflammatory cytokines, along with several other related molecules, mediate the inflammatory response associated with the immunological recognition of infectious agents. However, elevated levels of proinflammatory cytokines are also associated with a number of diseases of autoimmunity such as toxic shock syndrome, rheumatoid arthritis, osteoarthritis, diabetes and inflammatory bowel disease (Dinarello, C. A., etal., 1984, Rev. Infect. Disease 6 :51). In these diseases, chronic elevation of inflammation exacerbates or causes much of the pathophysiology observed. For example, rheumatoid synovial tissue becomes invaded with inflammatory cells which results in the destruction of cartilage and bone. An important and accepted therapeutic approach for potential drug intervention in these inflammatory diseases is the reduction of proinflammatory cytokines, such as TNFα and IL-13. A number of anti-cytokine therapies are currently in clinical trials. Efficacy has been demonstrated with a monoclonal antibody directed against TNFα in a number of autoimmune diseases, including the treatment of rheumatoid arthritis, Crohn's disease and ulcerative colitis (Rankin, E. C. C., et al., 1997, British J. Rheum. 35 : 334-342 and Stack, W. A., et al., 1997, Lancet 349 : 521-524).

Other anti-inflammatory therapies include nonsteroidal anti-inflammatory drugs (NSAIDs) which are medicines that relieve pain, swelling, stiffness, and inflammation by reducing the production of a variety of proinflammatory mediators, such as cytokines and prostaglandins. NSAIDs are associated with a number of side effects. The frequency of side effects varies between the drugs. The most common side effects are nausea, vomiting, diarrhea, constipation, decreased appetite, rash, dizziness, headache, and drowsiness. NSAIDs may also cause fluid retention, leading to edema. The most serious side effects are kidney failure, liver failure, ulcers and prolonged bleeding after an injury or surgery. Some individuals are allergic to NSAIDs and may develop shortness of breath when an NSAID is administered. People with asthma are at a higher risk for experiencing serious allergic reaction to NSAIDs.

It is an object of the present invention to provide alternative methods and compositions for the prevention and treatment of inflammatory diseases. In particular, it is an object to provide novel compositions capable of preventing or reducing the symptoms associated with asthma and inflammatory bowel disease (IBD), such as ulcerative colitis. Surprisingly, the objects set are achieved by the discovery that inositol-containing phospholipids are suitably used to suppress T cell activation. The suppressive effect was observed to be exerted at different levels. The first level is through direct inhibition, i.e. on the T cell itself. Unless specifically stated otherwise, the term "T cell" is meant to include any type of cell which carries a T cell receptor. Thus, also comprised are cells other than classical T lymphocytes which have a T cell receptor, such as NK T cells. The second level at which inositol phospholipids can suppress T cell activation is indirectly by modulating the process of antigen presentation. It was found that inositol phospholipids are effective suppressors of antigen presentation by antigen presenting cells. The effect was not limited to a specific antigen or type of antigen. For example, antigen presentation by MHC class I, II and CD1 molecules was inhibited.

The invention therefore relates to a composition for the prevention or treatment of a condition associated with unwanted T cell activation comprising as an active ingredient an inositol phospholipid, herein abbreviated as "IPL". The composition is for example a pharmaceutical composition or a food product with immunomodulatory properties. In eukaryotic cells, inositol phospholipids are quantitatively minor but vital components of membrane lipids with critical structural, metabolic and signalling roles. Thus, in contrast to known immunosuppressive agents, inositol phospholipids are natural constituents of the body. Accordingly, the risk of unwanted side effects of a composition of the invention is lower compared to the 'foreign' immunomodulators mentioned above.

Other aspects of the invention relate to the use of an IPL for the manufacture of a medicament for the treatment of any disorder associated with unwanted or excessive T cell activation, such as immune disease, allergic disorder or chronic inflammatory disease. It also relates to methods for suppressing the immune system of a subject, comprising administering a suitable dose of an IPL to said subject. The invention is of particular use for the treatment of asthma, diabetes Type I, rheumatoid arthritis (RA) and inflammatory bowel disease (IBD). Furthermore, the invention provides a food item or food supplement having immunomodulatory properties.

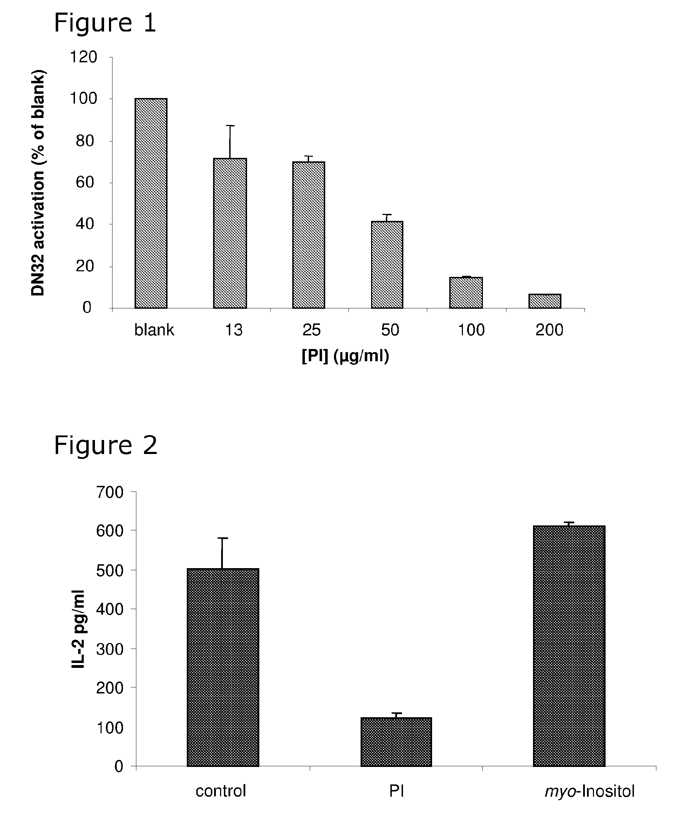

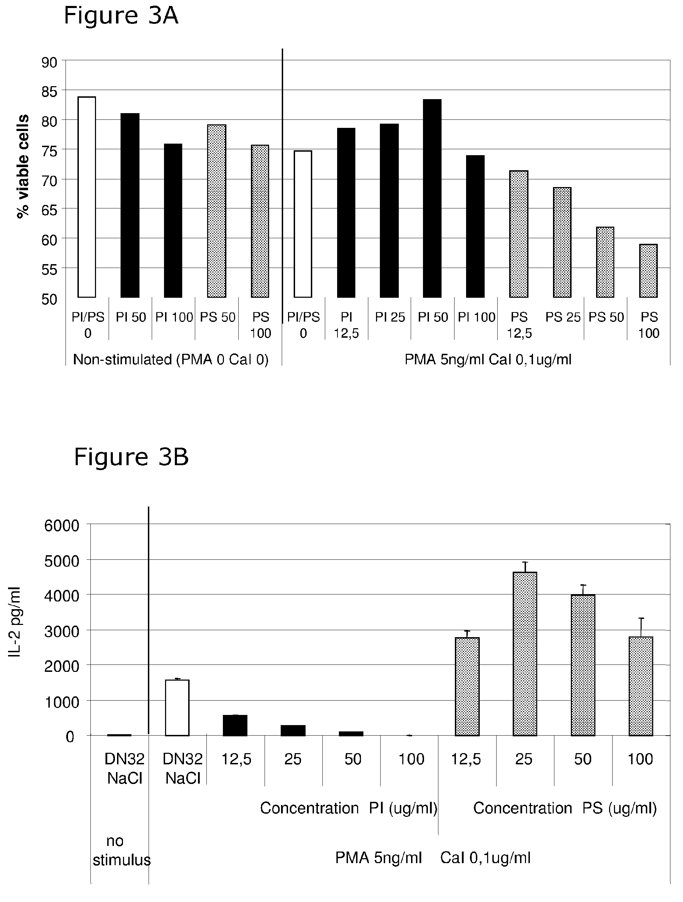

As shown herein, IPL is capable of blocking or modifying *in vitro* activation of T cells and NKT cells by both peptide-antigen presenting molecules (e.g. MHC class I, class II) as well as lipid-antigen presenting molecules (e.g. CD1) (see Figure 1). Furthermore, it was found that IPL can directly inhibit T cell activation induced by phorbol ester and calcium ionophore to mimic downstream T cell receptor signalling as well as by stimulation of the T cell receptor-complex (CD3) and co-stimulatory molecule CD28 (Fig. 3B). Importantly, the mucosal administration of IPL was very efficient to reduce or prevent the symptoms associated with asthma and ulcerative colitis in established animal models. The above effects were found to be dose-dependent. The presence of a phosphodiester bridge and a lipid moiety appear crucial for these effect, since myo-inositol had no significant suppressive effect (Figure 2).

Accordingly, a composition comprising an inositol phospholipid, such as PI, is suitably used to suppress or inhibit the activation of T cells and therewith suppress the unwanted effects of T cell activation, in particular cytokine production. A composition is advantageously used for the treatment or prevention of a condition that is associated with or caused by unwanted (e.g. excessive) activation of cells bearing a T cell receptor, like T lymphocytes or NKT cells. For example, a condition associated with unwanted T cell activation by an antigen-presenting molecule, either a peptide or lipid antigen-presenting molecule, can be ameliorated by a treatment with a composition comprising an IPL. In a specific embodiment, said condition is an MHC-associated condition. MHC class II molecules play a central role in the pathophysiology of several pulmonary diseases including asthma, sarcoidosis, hypersensitivity pneumonitis, and lung transplant rejection. Other MHC-associated diseases include Type I diabetes Mellitus, Crohn's disease, ulcerative colitis, Rheumatoid arthritis, Multiple Sclerosis and solid organ transplant rejections (such as liver and kidney). However, as is clear from the *in vitro* experiments described below, the immunosupressive effects of IPLs are not restricted to processes involving antigen-presenting molecules. Rather, they extend to the direct modulation of downstream T cell signalling, including the induction of proliferation and cytokine production. In one embodiment, the invention provides a composition comprising an IPL for the suppression of T cell activation, wherein said composition modulates an intracellular T cell event that requires *de novo* protein synthesis

*In vitro* studies showed that the pleiotropic suppressive effects of IPL were not due to an overall loss of cell viability of lipid-treated cells. Prolonged exposure of quiescent or activated cells to IPL did not induce cell death (Fig 3A). Moreover, the suppressive effect of IPL was found to be reversible, indicating that it does not cause permanent inactivation (data not shown). Thus, the invention provides methods for (reversibly) modulating the immune system at the level of antigen presenting cells as well as at the level of T cells. It is surprising that inositol phospholipids can block or modify distinct types of antigen-presenting molecules, i.e. peptide- and lipid-antigen presenting molecules having many different antigens with considerable structural variation. Although it cannot be excluded, it is thus unlikely that the effects disclosed herein are merely based on a competitive effect of inositol phospholipids for the antigen binding site. Without wishing to be bound by any theory, it is conceivable that the pleiotropic effect of inositol phospholipids is, among others, exerted at a level which the different antigen-processing molecules have in common, for example intracellular transport of the antigen-presenting molecules to the cell surface. More specifically, it is postulated herein that one mode of action of inositol phospholipids is based on interference with the intracellular trafficking of antigen-loaded molecules. Herewith, the invention also provides a method and a composition for modulating the transport of a molecule to the plasma membrane, preferably the endosomal transport, more preferably late endosomal transport to the plasma membrane. Said molecule may be an immunomodulatory molecule, for example MHC class I or II, a CD1 family member, or any other molecule that has to be transported to the plasma membrane, for example a transmembrane protein or a protein or peptide physically associated therewith such as a Tol-like receptor, for example TLR4.

The effects of IPL are specific for the inositol headgroup. The same dose of phosphatidyl serine (PS), also an acidic phospholipid, was not only found to be ineffective in suppressing T cell activation but even enhances PMA/Ca-induced IL-2 production (Fig. 3B). What is more, whereas PI does not affect cellular viability up to the highest dose tested (100 µg/ml), PS appears toxic to activated T cells in a dose-dependent fashion (see Figure 3A). This is in contrast to earlier studies on the immunomodulatory effects of acidic phospholipids. For example, Ponzin et al. (Immunopharmacology. 1989 Nov-Dec; 18(3): 167-76.) disclose immunosuppressive effects of intravenously administered PS vesicles in mice. Along that same line, Caselli et al. (Immunopharmacology. 1992 May-Jun;23(3):205-213) reported the inhibition of DNA synthesis in activated peripheral blood cells by PS. As PI was only partially effective (and even caused a stimulation of DNA synthesis at low concentrations), the effect was contributed to the phosphorylserine headgroup. Furthermore, only PS species containing unsaturated fatty acyl chains were active.

The term "inositol phospholipid" (IPL) as used herein refers to a conjugate of a lipid moiety and myo-D-inositol linked by a phosphodiester bridge. The inositol (hexahydroxycyclohexane) moiety may be phosphorylated. The lipid can be a glycerolipid or a sphingo phospholipid. A glycerolipid has a glycerol moiety as backbone whereas a sphingolipid has a sphingoid base as backbone. The fatty acyl chain length and degree of unsaturation can vary. It also refers to inositol phospholipid precursors (see further below) and acceptable salts of inositol phospholipids.

Inositol phospholipids for use in the present invention can be obtained from natural, synthetic or semi-synthetic sources. Animal (e.g. bovine) organs rich in inositol phospholipids such as brain and liver can be used as natural sources. Methods to isolate inositol phospholipids from natural sources are known in the art. For example, neomycin affinity chromatography can be used. IPL can be obtained from a complex phospholipid mixture what is commercially called lecithin. It is important to note that this mixture is actually only one-third true lecithin. Biochemically speaking, lecithin belongs to a group of nutrients known as lipids (fats, oils, waxes) and is a phospholipid called phosphatidylcholine. The other two-thirds of commercial lecithin is made up of other phospholipids, including phosphatidyl ethanolamine (PE), phosphatidyl serine (PS) and phosphatidyl inositol (PI).

In another embodiment, synthetic IPL is used. Methods for the synthesis of inositol phospholipids are known in the art (see for instance US 6,737,536 and references cited therein). US 6,737,536 discloses a method for the synthesis of inositol phospholipids, particularly phosphatidyl-myo-inositols, ceramide-phosphoinositols and their structural and stereochemical analogues. The published method is suitably used for laboratory scale preparation as well as for large scale industrial production.

In one embodiment, the IPL is a glycerophospholipid, for instance phosphatidyl-myo-inositol (abbreviated to PtdIns or PI) or a phosphorylated derivative thereof, such as PIP, PIP₂ or PIP₃. Phosphatidylinositol is an important lipid, both as a key membrane constituent and as a participant in essential metabolic processes in plants and animals (and in some bacteria). It is an acidic (anionic) phospholipid that in essence consists of a phosphatidic acid backbone, linked via the phosphate group to inositol. In most organisms, the stereochemical form of inositol is myo-D-inositol (with one axial hydroxyl), although other forms (scyllo- and chiro-) have been found on occasion in plants.

Preferably, the inositol moiety of the IPL is in the 1D-1-myo-inositol configuration and the glycerol moiety is in the sn-glycero-3-phospho configuration. The fatty acyl chains at the sn-1 and sn-2 position of the glycerol backbone of the glycerolipid will generally be ester-linked. The glycero residue can be esterified with mixtures of saturated long carbon chain fatty acyls, and unsaturated and polyunsaturated fatty acyls collectively referred to as (poly)unsaturated fatty acyls. A composition preferably comprises at least a diacylphosphatidylinositol.

IPL from natural sources is typically a complex mixture of molecular species, differing each in their fatty acyl composition and/or distribution between the glycero-1 and -2 positions. The fatty acid composition of phosphatidylinositol is rather distinctive. In animal tissues, the characteristic feature is a high content of stearic (C18:0) and arachidonic acids (C20:4). All the stearic acid is linked to position sn-1 and all the arachidonic acid to position sn-2, and as much as up to 80% of the total lipid may consist of the single molecular species sn-1-stearoyl-sn-2-arachidonoyl-glycerophosphorylinositol. In plant phosphatidylinositol, palmitic acid (C16:0) is the main saturated fatty acid while linoleic (C18:1) and linolenic (C18:2) acids are the main unsaturated components. Again, much of the saturated fatty acids are in position sn-1 and the unsaturated in position sn-2. However, the sn-1-fatty acyl residue may also be replaced by *O*-alkyl or O-alkenyl to yield alkyl-acyl PI or alkenyl-acyl PI, respectively. Also comprised are lyso-inositolphospholipids which lack the 2-fatty acyl, and thus have a free hydroxyl group at the glycero-2 position.

In another embodiment, a composition comprises the sphingophospholipid ceramide-phosphoinositol (CerPhosIns) or a derivative thereof. Preferably, the sphingosine/ceramide residue has the D-erythro stereochemical configuration as in naturally occurring CerPhosIns. CerPhosIns can contain variable chain length and degree of unsaturation in the two alkyl groups of the ceramide residue, and may carry additional unsaturation, hydroxyl or related functional groups. Also encompassed by the present invention are the sphingosyl-phosphoinositols (Sphingosyl-PhosIns) which lack the amide fatty acyl and have a free 2-amino group which may be derivatized further. A composition may also comprise a combination of two or more distinct inositol phospholipids. It may for instance comprise a mixture of IPLs which are distinct with respect to the lipid backbone (e.g. glycerol or ceramide), the fatty acyl composition, the type of fatty acyl linkage (e.g. ester or ether), and/or the inositol headgroup (e.g. carrying zero, one, two or three phosphates). Furthermore, other lipids, be it a phospholipid or a neutral lipid, like diglycerides or sterols, may be present in addition to the IPL. In a preferred embodiment, the IPL of mixture of IPLs makes up at least 50%, preferably at least 75%, more preferably at least 90%, most preferably at least 95% or even 99 or 100% of the total lipid content of the composition.

Preferably, e.g. for economical reasons, the IPL for practicing the invention is derived from a natural source, for example from plant or animal tissue. Other organisms may be used as well. Tissue-derived IPLs contain a mixture of various molecular species of inositol phospholipids, in particular with respect to the fatty acid composition. In a preferred embodiment, the ratio of saturated / unsaturated fatty acids in (tissue-derived) inositol phospholipid is at least 1.0, more preferably at least 1.1. IPL with a fatty acid content of more than 40% stearoyl (C18:0) is preferred, optionally in combination with at least 10% arachidonic acid (C20:4). A very good natural source of IPL for use in the invention is mammalian liver, for example bovine liver. Other useful sources include heart.

The polar inositol headgroup of the IPL is preferably not conjugated to a moiety other than to one or more phosphate groups. For example, the IPL is preferably free of saccharides glycosidically linked to the inositol moiety, unlike as is observed for immunogenic components of bacterial cell walls including acyl glyceryl phosphatidylinositol manno-oligosaccharide (PIM). In addition, for the pharmaceutical composition to be of therapeutic or prophylactic value, it is not necessary that the IPL is bonded or conjugated to a carrier moiety, for example to a physiologically acceptable monomer, dimer, oligomer or polymer as disclosed in US2004/0229842.
Conditions that would benefit from a composition comprising an IPL include any condition in which attenuation of antigen-presenting molecules and/or excessive or unwanted (natural killer) T cell activation is desirable. In one embodiment, a composition of the invention is used for the treatment or prophylaxis of a condition in which attenuation of NKT or (autoreactive) T cell activation is desirable. Natural killer T (NKT) cells are a subset of T cells that share properties of natural killer cells and conventional T cells. They are involved in immediate immune responses, tumour rejection, immune surveillance and control of autoimmune diseases. Most NKT cells express both an invariant T cell antigen receptor and the NK cell receptor NK1.1, and are referred to as invariant NKT cells. This invariant T cell receptor is restricted to interactions with glycolipids presented by the non-classical MHC, CD1d. These NKT cells rapidly produce high levels of interleukin (IL)-2, IFN-gamma, TNF-alpha, and IL-4 upon stimulation through their TCR. Most also have cytotoxic activity similar to NK cells. NKT cells are involved in a number of pathological conditions, and have been shown to regulate viral infections in *vivo*, and control tumor growth. They may also play both protective and harmful roles in the progression of certain autoimmune diseases, such as diabetes, lupus, atherosclerosis, ulcerative colitis and allergen-induced asthma.

Preferably, the condition is ameliorated by attenuation or suppression of peptide-antigen presentation, in particular by MHC class I or II molecules. Such a condition can for instance be an auto-immune disease, an allergic disorder, a transplant rejection or a (chronic) inflammatory disease. Preferably, the disease or symptoms associated therewith allow topical treatment with a composition of the invention, more preferably by mucosal administration. However, systemic administration of an IPL-containing composition can also be envisaged.

Autoimmune diseases arise from an overactive immune response of the body against substances and tissues normally present in the body. Examples of auto-immune diseases are: systemic lupus erythematosus (SLE), rheumatoid arthritis (RA), post-partum thyroid dysfunction, auto-immune thromocytopenia, psoriasis, scleroderma, dermatitis herpetiformis, polymyositis, dermatomyositis, pemphigus vulgaris, spondyloarthropathies such as ankylosing spondylitis, vitiligo, Sjögren's syndrome, multiple sclerosis (MS), Crohn's disease, myasthenia gravis, ulcerative colitis, autoimmune neuropathies such as Guillain-Barré, primary biliary cirrhosis, autoimmune hepatitis, autoimmune uveitis, Type 1 or immune-mediated diabetes mellitus (DM1), autoimmune hemolytic anemia, pernicious anemia, Grave's disease, autoimmune thrombocytopenia, Hashimoto's thyroiditis, autoimmune oophoritis and orchitis, autoimmune disease of the adrenal gland, Anti-phospholipid syndrome, vasculitides such as Wegener's granulomatosis and Behcet's disease.

Allergic disorders occur when the immune system overreacts to exposure to antigens in the environment. The substances (antigens) that provoke such attacks are called allergens. The immune response can cause symptoms such as swelling, watery eyes, and sneezing, and even a life-threatening reaction called anaphylaxis. Taking medications called antihistamines can relieve most symptoms. Some examples of allergic disorders are asthma and eczema. Asthma is a respiratory disorder that can cause breathing problems, frequently involves an allergic response by the lungs. If the lungs are oversensitive to certain allergens (like pollen, molds, animal dander, or dust mites), it can trigger breathing tubes in the lungs to become narrowed, leading to reduced airflow and making it hard for a person to breathe. Eczema is a scaly, itchy rash also known as atopic dermatitis. Environmental allergies (to dust mites, for example), seasonal allergies (such as hay fever), drug allergies (reactions to specific medications or drugs), food allergies (such as to nuts), and allergies to toxins (bee stings, for example) are the common conditions people usually refer to as allergies.

The present inventors investigated the *in*, vivo anti-inflammatory effects of IPLs in two different animal models: a hapten-induced colitis model, and an ovalbumin-induced asthma model, representing valid murine model systems for human Inflammatory Bowel Diseases, and human allergic asthma, respectively.

The oxazolone colitis model (Boirivant, Exp. Med., Volume 188, Number 10, November 16, 1998 1929-1939) is induced in SJL/J mice by the rectal instillation of the haptenating agent, oxazolone, and is characterized by a rapidly developing colitis confined to the distal half of the colon; it consists of a mixed neutrophil/lymphocyte infiltration limited to the superficial layer of the mucosa which is associated with ulceration. Oxazolone colitis is a T helper cell type 2 (Th2)-mediated process since stimulated T cells from lesional tissue produce markedly increased amounts of interleukin (IL)-4 and IL-5 (see Nieuwenhuis et al. "Disruption of T helper 2-immune responses in Epstein-Barr virus-induced gene 3-deficient mice". Proc Natl Acad Sci U S A. 2002 Dec 24;99(26): 16951-6; and Heller et al. "Oxazolone colitis, a Th2 colitis model resembling ulcerative colitis, is mediated by IL-13-producing NK-T cells." Immunity. 2002 Nov;17(5):629-38). The histologic features and distribution of oxazolone colitis have characteristics that resemble IBD, specifically ulcerative colitis (UC) and thus sharply distinguish this model from many other models such as acetic-acid induced colitis in rats, which is regarded as a damage model that is T cell independent

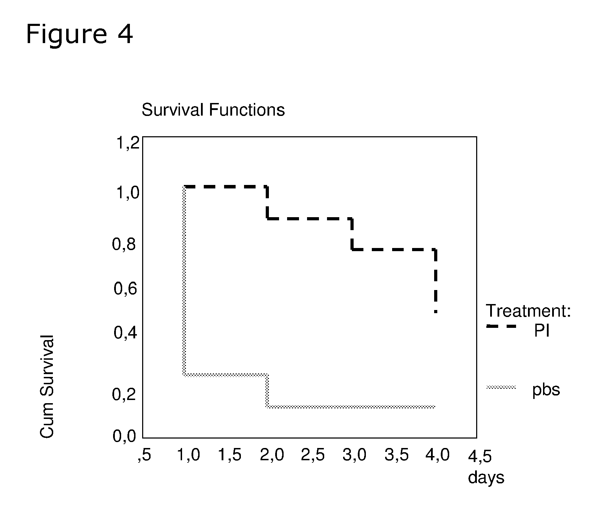

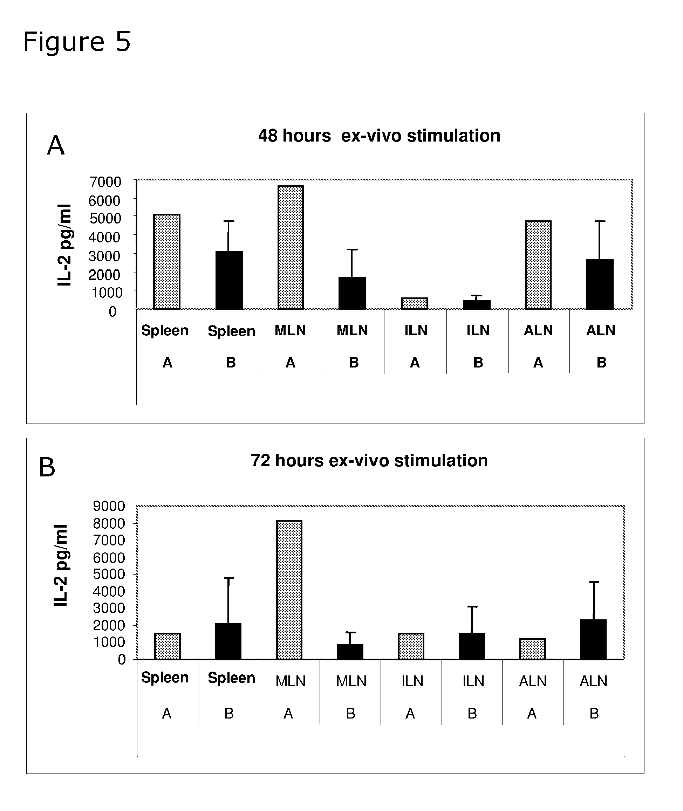

The *in vivo* immunosuppressive action of inositol phospholipids was investigated in oxazolone colitis, a mouse model for ulcerative colitis, an inflammatory bowel disease (IBD). For details, see Example 2A. In short, mice sensitized subcutaneously with oxazolone were challenged by rectal administration of oxazolone together with a varying dosage of inositol phospholipid. Strikingly, mice which had received IPL showed higher early survival rates (Fig. 4). Furthermore, cytokine production by intestinal T cells upon *ex vivo* activation was strongly suppressed in cells obtained from IPL-treated mice (Fig. 5) .

The murine OVA-inhalation model for human allergic asthma used by the present inventors is also well established and is described for example in Hessel et al. (Eur J Pharmacol. 1995 Dec 7;293(4):401-12) and Hofstra et al. (J. Immunol. 161:5054). Briefly, BALB/c mice are sensitized to ovalbumin (OVA) by intraperitoneal (IP) injections and subsequently challenged with aerosolized OVA (days 13 and 14). Some mice were exposed intratracheally with 0, 200 or 2000 µg/ml IPL using an aerosol spray before undergoing OVA aerosol challenge. A control group was exposed to OVA alone.Thereafter, mice were sacrificed.

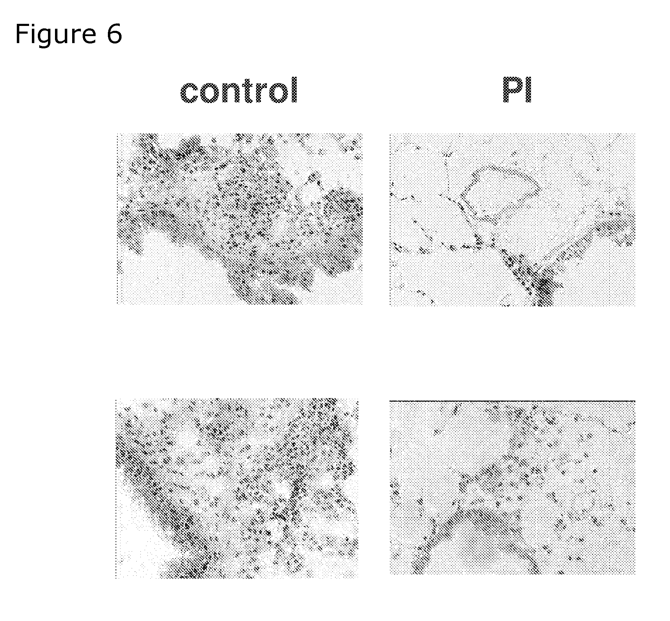

As exemplified in more detail below (Example 2B), a composition comprising as an active ingredient at least one IPL showed pronounced immunosuppressive effects in the asthma animal model. Histological analysis of stained lung sections showed that PI treatment prevented the typical histo-pathological changes associated with asthma (Fig. 6).

In one aspect the invention provides a pharmaceutical composition comprising as an active ingredient at least one IPL, said composition being adapted or formulated for pulmonary administration. Also provided is the use of an IPL, in particular PI, for the manufacture of a medicament for the treatment or prevention of asthmatic conditions in a mammal, preferably in a human subject. Phospholipids, including IPL, have been used before in pulmonary compositions to facilitate the delivery of active agents. Typically the lipid is incorporated in liposomes or other type of lipid body allowing for the delivery and dispersion of proteinaceous active agents. WO2005/055994 for example discloses a composition comprising a surfactant mixture comprising phospholipids and a lung-surfactant polypeptide of a specific amino acid sequence that maximizes its interaction with the alveoli. In contrast, the antiasthma composition of the present invention comprises IPL rather than a lung-surfactant polypeptide as active agent. Accordingly, in one embodiment the composition for the pulmonary administration of IPL does not contain a lung-surfactant polypeptide or other kind of proteinaceous therapeutic molecule.

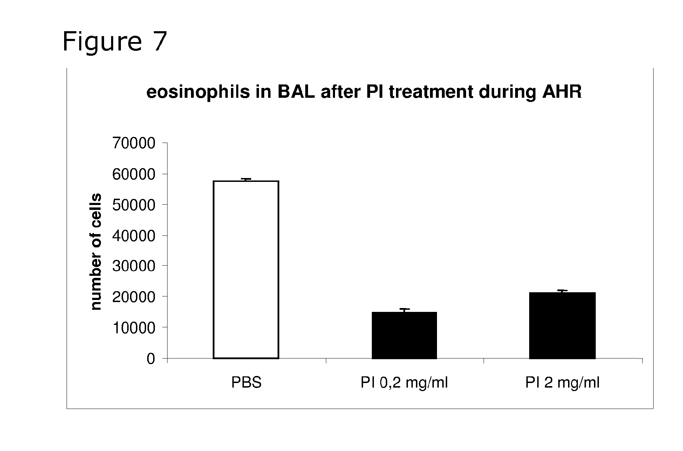

Surprisingly, analysis of broncho alveolar lavages (BAL) of the OVA-challenged mice revealed a strong suppressive effect of PI on eosinophilia (Fig. 7). Eosinophilia refers to conditions in which abnormally high amounts of eosinophils are found in either the blood or in body tissues. Eosinophilia occurs in a wide range of conditions, including allergic diseases such as asthma, hay fever and eosinophilic esophagitis. The invention therefore also relates to methods and compositions for preventing or treating eosinophilia.

Worldwide the main cause of eosinophilia is parasitic infection. It can also occur in relation to common skin diseases, medicine reactions, and parasitic infections. Increased numbers of eosinophils are associated with allergic disease or parasitic infections. This is helpful in combating parasitic infections but in cases of allergic diseases as they accumulate in tissues and cause damage. For example, in asthma, eosinophilia causes damage to the airways of the lung.

The exact mechanism by which IPL exerts its suppressive effects in the asthma mouse model is at present unclear. Most likely, it is the combined action on antigen-presentation, e.g. by the MHC and CD1 molecules, and on blocking downstream TCR signaling per se, irrespective of how the TCR is triggered. It has been proposed that CD1 plays an important role in asthma since mice lacking CD1d or NKT cells showed a reduced immune response when challenged by inhalation exposure of OVA antigen ((Akbari et al. Nature Med.2003 May;9(5):582-8. ) However, the suppressive effect observed of the IPL as disclosed herein is much stronger than observed in the CD1d-deficient mice. These data indicate that effect of inositol phospholipids cannot be explained (solely) by a dysfunctional lipid-antigen presentation by CD1 but that it has a much broader spectrum of action. Dendritic cells (DCs) are the primary antigen presenting cells (APCs) responsible for the capture of allergens in the airways and the shuttling of processed allergens to the draining lymph nodes where antigen presentation and T cell activation take place. In one embodiment, the invention provides the use of inositol phospholipids for the suppression of antigen presentation by DCs.

A pharmaceutical composition comprising an inositol phospholipid or a pharmaceutically acceptable salt (e.g. sodium or ammonium) thereof may be in the form of a capsule, tablet, lozenge, enema, dragee, pill, droplet, suppository, powder, spray, vaccine, ointment, paste, cream, inhalant, patch, aerosol, and the like. Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

As pharmaceutically acceptable carrier, any solvent, diluent or other liquid vehicle, dispersion or suspension aid, surface active agent, isotonic agent, thickening or emulsifying agent, preservative, encapsulating agent, solid binder or lubricant can be used which is most suited for a particular dosage form and which is compatible with the inositol phospholipid.

Pharmaceutically acceptable carriers in therapeutic compositions may contain liquids such as water, saline, glycerol and ethanol. Saline is preferred. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared.

For therapeutic treatment, inositol phospholipids are applied to the subject in need thereof. The inositol phospholipid may be administered to a subject by any suitable route, preferably in the form of a pharmaceutical composition adapted to such a route and in a dosage that is effective for the intended treatment. A mucosal route of administration, e.g. intrarectal or intratracheal, is preferred as this reduces the risk of unwanted side-effects when compared to systemic administration. Therapeutically effective dosages of the inositol phospholipid required for treating the disorder, for instance for prevention and/or treatment of a disorder selected from the group consisting of asthma, rheumatoid arthritis, diabetes and inflammatory bowel disease in the body of a human or animal subject, can easily be determined by the skilled person, for instance by using animal models. Of course, IPLs can also be used in combination with one or more immunosuppressant or anti-inflammatory (drug) therapies known in the art, including corticosteroids and nonsteroidal anti-inflammatory drugs (NSAIDs).

The term "therapeutically effective amount" as used herein refers to an amount of IPL to reduce or prevent unwanted or excessive T cell activation, or to exhibit a detectable therapeutic or prophylactic effect. The effect can be detected by, for example, measurement of cytokine production or by any other suitable method of assessing the progress or severity of inflammatory responses which methods are known per se to the skilled person. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgment of the clinician or experimenter. Specifically, the compositions of the present invention can be used to reduce or prevent asthma or ulcerative colitis (UC) and/or accompanying biological or physical manifestations. Methods that permit the clinician to establish initial dosages are known in the art. The dosages to be administered must be safe and efficacious.

For purposes of the present invention, an effective daily dose will be from about 0.01 µg/kg to 1 g/kg and preferably from about 0.5 µg/kg to about 400 mg/kg of the inositol phospholipid in the individual to which it is administered.

Yet in another alternative embodiment, the inositol phospholipid or compositions of the invention may be administered from a controlled or sustained release matrix inserted in the body of the subject.

A composition wherein said at least one IPL makes up less than 50 percent, preferably less than 30 percent, more preferably less than 25 percent of the dry weight of the composition was found to be effective in reducing or preventing inflammatory symptoms. Given the broad spectrum of immunomodulatory effects of inositol phospholipids as disclosed herein, the invention also relates to a food item, food supplement or food additive comprising an IPL. The food item can be a solid or a liquid food product. The food additive is defined as a composition that can be used for the manufacture of a (functional) food item. A food supplement is defined as a composition that can be consumed in addition to the normal food intake and which comprises elements or components that are not or in only minor amounts, present in the normal diet and of which sufficient or increased consumption is desired. The composition of a food item does not necessarily differ much from that of a food supplement or food additive.

For a food composition of the invention, the same preferences exist with respect to the type of IPL. An IPL-enriched food item or food supplement as described above may suitably comprise 0.01 to 99.9 wt.% of an inositol phospholipid. In a preferred embodiment such a food item or food supplement comprises from 0.01 to 50 wt.%, preferably from 0.01 to 10 wt.%, more preferably from 0.01 tot 5 wt.% of an inositol phospholipid, a precursor or acceptable salt thereof.

In order to make a food item or food supplement comprising an elevated amount of an IPL suitable for human or animal consumption, the nutritional value, texture, taste or smell may be improved by adding various compounds to said item or supplement. The skilled person is well aware of the different sources of protein, carbohydrate and fat that may be used in food items or food supplements according to the invention and of the possible sweeteners, vitamins, minerals, electrolytes, coloring agents, odorants, flavoring agents, spices, fillers, emulsifiers, stabilizers, preservatives, antioxidants, food fibers, and other components for food items that may be added to improve its nutritional value, taste or texture. The choice for such components is a matter of formulation, design and preference. The amount of such components and substances that can be added is known to the skilled person, wherein the choice may e.g. be guided by recommended daily allowance dosages (RDA dosages) for children and adults and animals.

Portions for intake of the food item or food supplement may vary in size and are not limited to the values corresponding to the recommended dosages. The term "food supplement" is herein not intended to be limited to a specific weight or dosage.

A composition of a food item or food supplement as described above may in principle take any form suited for consumption by man or animal. In one embodiment the composition is in the form of a dry powder that can be suspended, dispersed, emulsified or dissolved in an aqueous liquid such as water, coffee, tea, milk, yogurt, stock or fruit juice and alcoholic drinks. To this end, the powder may be provided in unit-dosage form.

In an alternative preferred embodiment an IPL-comprising composition in the form of a dry powder is tabletted. To that end, a composition for a food supplement according to the invention may very suitably be provided with fillers, such as microcrystalline cellulose (MCC) and mannitol, binders such as hydroxypropylcellulose (HPC), and lubricants such as stearic acid or other excipients.

A composition of a food item or food supplement as described above may also be provided in the form of a liquid preparation wherein the solids are suspended, dispersed or emulsified in an aqueous liquid. Such a composition may be admixed directly through a food item or may e.g. be extruded and processed to grains or other shapes.

In an alternative embodiment a food item or food supplement may take the shape of a solid, semi-solid or liquid food item, such as a bread, a bar, a cookie or a sandwich, or as a spread, sauce, butter, margarine, dairy product, and the like. Preferably, an IPL is applied in a dairy product, such as for instance a butter or margarine, custard, yogurt, cheese, spread, drink, or pudding or other dessert. Preferably the food item is a drink, as one can easily estimate the intake of lipid this way. The IPL can also be used in butters or fats used for frying and baking, because they are relatively resistant to degradation at high temperatures. This characteristic also enables use of the IPL in food items or food supplements that undergo a pasteurization or sterilization treatment. IPL-containing diet or slimming products also constitute preferred embodiments of food items or food supplements according to the invention.

If a food item according to the invention is used as an animal feed, the food item may e.g. be prepared in the form of a powder, a grain, a waffle, a porridge, a block, a pulp, a paste, a flake, a cook, a suspension or a syrup.

For administering to humans the food item of the invention may very suitably be prepared in the form of a food supplement.

The present invention further relates to a method for the preparation of a food item or food supplement according to the invention, comprising enriching a food item or food supplement with an inositol phospholipids, a precursor, or an acceptable salt thereof.

In one embodiment the invention provides a method for the preparation of a food item or food supplement enriched with an IPL, comprising processing an inositol phospholipid in a food item or food supplement, preferably to an amount of 0.01 to 99.9 wt.%, more preferably to an amount of from 0.01 to 50 wt.%, even more preferably to an amount of from 0.01 tot 10 wt.%, and most preferably to an amount of from 0.01 tot 5 wt.%. The actual amount of IPL incorporated in a food item according to the invention depends on the type of lipid(s) and its use and the skilled person is capable of determining this amount in the context of the present disclosure.

In a method for preparing a food item according to the invention the food item may first be prepared separately and then be joined with an IPL to provide a food item according to the invention wherein said phospholipid is incorporated in the food item. The food item may be separately prepared by conventional methods such as by mixing, baking, frying, cooking, steaming or poaching and may, if necessary, be cooled prior to joining with the IPL. According to another suitable embodiment, the IPL is incorporated as a component in the food item during the preparation thereof.

A food item or food supplement according to the present invention may very suitably be defined as a nutraceutical composition. Nutraceuticals can be defined as natural products that are used to supplement the diet by increasing the total dietary intake of important nutrients. This definition includes nutritional supplements such as vitamins, minerals, herbal extracts, antioxidants, amino acids, and protein supplements. Nutraceutical products fit into the newly created product category of "Dietary Supplements" as established by the F.D.A. in the Dietary Supplement Act of 1994. This act specifically defined dietary supplements to include: vitamins, minerals, herbs or other botanicals, antioxidants, amino acids, or other dietary substances used to supplement the diet by increasing the total daily intake.

A "nutraceutical composition" is defined herein as a food composition fortified with ingredients capable of producing health benefits. Such a composition in the context of the present invention may also be indicated as foods for special dietary use; medical foods; and dietary supplements. The food item and/or food supplement of the present invention is a nutraceutical composition since it is fortified with an IPL according to the invention and since it is capable of treating or preventing diseases or conditions associated with excessive or unwanted T cell activation. For example, the food item or supplement may help to prevent or reduce symptoms associated with an inflammatory condition such as allergies (e.g. hay fever) and the like.

As with the pharmaceutical composition, the amount of IPL in the food or food additive will depend on several factors. The food product will generally comprise a concentration of IPL that is sufficient to provide a consumer with an effective amount of lipid upon consumption of a regular (e.g. daily) portion of the food product. For example, the food product may be a beverage which is typically consumed as a 200-ml portion wherein the inositol phospholipid is present in an amount of 0.0005 to 2 mg per liter, preferably etc. In another embodiment, the invention provides a spread comprising IPL.

It will be recognized by those skilled in the art that the optimal quantity and spacing of individual dosages for achieving the therapeutic effects of the pharmaceutical composition, food item or food supplement described herein may easily be determined by the skilled person. They will be determined by the nature and extent of the condition being treated, as well as by the form, route and site of administration, and the particular individual undergoing treatment, and that such optima can be determined by conventional techniques. Typically, the treatment will involve the administration of inositol phospholipid in amount of approximately 0.001 to 1 gram , preferably 0.005 to 0.5 gram, more preferably 0.01 to 0.1 gram per kilogram of body weight per 24 hours. It will also be appreciated by those skilled in the art that the optimal dosage regimen, i.e. the number of doses, can be ascertained using conventional course of treatment determination tests well known in the art. The present inventors observed that mice that received 200 µl of PI 2 mg/ml (i.e. 400 µg) intraveneously did not show any symptoms or adverse signs during a 3 hour observation period post-administration. Generally speaking, a therapeutic dosing regimen will involve the administration of the selected dosage formulation comprising an inositol phospholipid, e.g. a PI aerosol spray, at least once daily, preferably one to four times daily, until the symptoms have subsided. Or given in times of remission, in order to prevent a disease flare-up (exacerbation). For purposes of the present invention, an effective dose will be from about 0.01-5% of the dry food weight in the individual to which it is administered, meaning that for an adult human being the daily dose will be between about 0.04 and 35 grams of inositol phospholipid.

Preferably, a pharmaceutical composition is formulated such that it is suitable for the topical mucosal administration to a subject in need thereof, e.g. a human or animal subject suffering from an immune disease. Mucosal routes of drug delivery include the mucosal linings of the nasal, rectal, vaginal, ocular and oral cavity. Sites for topical mucosal administration include the airway passage (e.g. nasal or intratracheal delivery) and the gastrointestinal tract. In one embodiment, the invention provides a spray comprising inositol phospholipid. In particular, it provides the use of an aerosol spray comprising inositol phosphilipid, e.g. PI, for the treatment or prevention of symptoms associated with asthma.

In another embodiment, it provides a rectal enema or suppository comprising an inositol phospholipid. These are particularly useful in the treatment or control of inflammatory bowel diseases. The two main categories of inflammatory bowel disease are ulcerative colitis and Crohn's disease. Ulcerative colitis is characterized by recurring episodes of inflammation of the mucosal layer of the large bowel not related to an intestinal infection. The inflammation involves the rectum and may extend proximally in a continuous fashion. Crohn's disease is characterized by recurring episodes of suppurative inflammation of any part of the bowel, from the mouth to the anus. This inflammation is transmural, and can result in strictures, microperforations, and fistulae. The inflammation is noncontiguous and thus can produce skip lesions throughout the bowel. A composition of the invention comprising an inositol phospholipid for use in the treatment of inflammatory bowel disease may of course also contain one or more therapeutic substances and excipients commonly used. For example, it may contain other anti-inflammatory drugs such as an aminosalicylate (e.g.5-ASA) or (cortico)steroid. In yet another embodiment, it provides an ointment comprising an IPL. These are particularly useful in the treatment or control of inflammatory diseases of the skin such as psoriasis.

A known issue relating to the administration of lipids to a subject, be it in foods or in pharmaceutical compositions, is that they can be metabolized, e.g. by general or specific lipases. This is particularly relevant for application of phospholipids in the digestive tract. Also within the present invention, metabolism (e.g. degradation or conversion) of an IPL administered to a subject may occur prior to the moment the lipid reaches its site of action, such as APCs and/or T cells. This issue may be addressed by administering an IPL or a pharmaceutically acceptable salt thereof, alone or in combination, as a so-called precursor compound which compound comprises certain substituents as a result of which the IPL can no longer, or only at reduced rates, be metabolized. These precursors are preferably resistant to hydrolysis in the upper parts of the digestive tract (e.g. mouth, stomach), and are for instance split relatively easy in the lower part of the digestive tract (e. g. coecum, colon), if the IPL should have its working especially there. Preferably, when the intake of the precursor is via the oral route, the intact or metabolized precursors are taken up into the blood stream and transported to the target site(s) where they may be activated in order to exert their beneficial effect. Thus, it is possible that activation occurs when the compound has been absorbed from the digestive tract, e.g. in the blood or the liver. As a result, the amount of the compound is raised at those locations where the sphingolipid has its action. For instance, an IPL precursor may be used that can be split or activated in vivo by a suitable enzyme so that the IPL is liberated that may suppress T cell activation in the subject.

An inositol phospholipid or a precursor or a pharmaceutically acceptable salt thereof, may be provided to a subject in need thereof for prophylactic or therapeutic reasons. An inositol phospholipids, a precursor or a pharmaceutically acceptable salt thereof, may be provided to a subject in need thereof in the form of a food item or food supplement, or in the form of a pharmaceutical preparation, all such administration forms being capable of preventing the development and/or to alleviate the symptoms of unwanted T cell activation. In particular, the development and/or severity of asthma, diabetes and inflammatory bowel disease are considered.

An IPL or a precursor, or a pharmaceutically acceptable salt thereof, may be used in a food item or food supplement. A food supplement is defined as a composition that can be consumed in addition to the normal food intake and which comprises elements or components that are not or in only minor amounts, present in the normal diet and of which sufficient or increased consumption is desired. The composition of a food item does not necessarily differ much from that of a food supplement.

A food item or food supplement as disclosed herein comprises an amount of inositol phospholipid that is higher than the amount that would normally or without human intervention occur or be found in said food item or food supplement. This elevated amount of an inositol phospholipid may arise through specific addition of said sphingolipid to a food item that does not normally comprise said sphingolipid in said elevated amount, i.e. by enrichment of the food item with said phospholipid. Alternatively, genetic engineering may be used to produce food items comprising said IPL in an elevated amount, for instance by engineering the biosynthetic routes for the production of such IPL in a plant, or yeast or other microorganism used for the production of a food item in such a way that said IPL is produced in said organism in an elevated amount.

A food item or food supplement with enhanced levels of an inositol phospholipid, or a precursor or a derivative thereof is advantageously used for the prevention and/or treatment of an inflammatory disease, in particular allergic asthma or ulcerative colitis. The food item or supplement also finds its use in a diet for treating and/or preventing an inflammatory disease, in particular allergic asthma or ulcerative colitis. Thus, the invention provides an immunosuppressive food item or food supplement comprising enhanced IPL levels, for example exogenously added PI, preferably mammalian PI, more preferably bovine liver PI or a synthetic analogue thereof.

Also provided herein is a method of preventing the occurrence of an inflammatory disease, in particular allergic asthma or ulcerative colitis, in a healthy subject comprising providing said subject a diet with enhanced levels of inositol phospholipid or a precursor, a derivative or a pharmaceutically acceptable salt thereof. Alternatively, or in addition, said subject may be administered (e.g. via the mucosal route) a pharmaceutical composition according to the invention. The invention furthermore provides the use of a pharmaceutical composition according to the invention for the prevention and/or treatment of an inflammatory disease, in particular allergic asthma or inflammatory bowel diseases.

A further aspect relates to a method of treatment of a subject suffering from an inflammatory disease, in particular allergic asthma or inflammatory bowel diseases, such as ulcerative colitis, said method comprising administrating to a subject in need thereof a therapeutically effective amount of a pharmaceutical composition according to the invention.

### LEGENDS TO THE FIGURES

**Figure 1****: Dose-dependent inhibition by PI of CD1d-restricted NKT cell activation**. A CD1d transfected epithelial cell line T84D was pulsed with alpha-GalactosylCeramide and co-cultured with PI at different concentrations. NKT cell activation was determined by measurement of IL-2 present in the supernatants.
**Figure 2****: Phosphatidyl-inositol but not myo-inositol blocks CD1d dependent NKT cell activation.** A CD1d transfected epithelial cell line T84D was pulsed with aGalactosylCeramide and co-cultured with either solvent control, PI or myo-inositol. NKT cell activation was determined by measurement of IL-2 present in the supernatants.
**Figure 3****: Differential effect of PI and PS on cellular viability and immune suppression**. T cells were suspended at 10⁶ cells/ml in 24-wells plates (1 ml suspension per well). Cells were either left untreated (PMA 0 Cal 0) or activated with 5 ng/ml PMA + 0.1 µg/ml calcium ionophore A23187 (PMA 5 ng/ml Cal 0.1 ug/ml) in the presence of PI or PS at the indicated concentrations (expressed in µg/ml). Panel A: Following a 24 hour incubation period in the presence of the indicated lipids and stimuli cellular viability was measured using flow cytometric detection of 7-AAD stained cells. PI does not significantly affect cellular viability, whereas PS is toxic for activated cells in a dose-dependent manner. Panel B : inhibitory effect of PI versus the stimulatory effect of PS on T cell activation measured by IL-2 cytokine production.
**Figure 4****: IPL strongly affects early survival in oxazolone colitis model.** For experimental details see Example 2A.
**Figure 5****: Suppression of IL-2 production by intestinal T cells upon IPL intrarectal treatment of oxazolone-colitis mice.** Mice were treated with saline (group A) or PI (group B) twenty minutes prior to challenge with oxazolone to induce colitis. Mice were sacrificed and intestinal T cells were analyzed for IL-2 production upon stimulation by aCD3/aCD28 during a period of 48 hours (panel A) or 72 hours *(panel B).* ALN = axillair LN; MLN = mediastinal LN; ILN = iliacal LN. For further details see Example 2A.
**Figure 6****: Treatment with IPL (non-liposomal aerosol) prevents the typical histo-pathological changes that are associated with an asthma mouse model.** For experimental details see Example 2B.
**Figure 7****: Treatment with PI (aerosol) prevents the eosinophilia that is associated with an asthma mouse model.** For experimental details see Example 2B.

### EXPERIMENTAL SECTION

The Examples 1 and 2 below demonstrate the immunosuppressive effects of inositol phospholipids under *in vitro* conditions, whereas Example 3 describes the *in vivo* effects of IPL in animal models for immune disorders. All *in vivo* experiments were performed in accordance with the ethics code for animal experimentation by the Experimental Animal Committee of Erasmus University Rotterdam, the Netherlands. Materials used include phosphatidyl inositol (PI) obtained from Sigma (Bovine liver PI; Sigma catalogue number P 8443), I-Inositol/myo-inositol obtained from Becton Dickinson.

### Example 1: NKT cell activation through CD1d lipid antigen presentation

On day 0, T84D cells (a human epithelial cell line transfected with CD1d) were incubated overnight with aGalCer (10 mg/ml). Varying doses of PI were added to T84D cells at varying time-points. Next, T84D cells were washed once with PBS and DN32 cells (a murine 1.1 positive NKT cell line) were added (T84D: DN32 = 1: 10) and co-cultured for 24 hours. Supernatants were collected and IL-2 cytokine production by DN32 (reflecting T cell activation) was determined by means of ELISA. The results are shown in Figure 1.

### Example 2: In vivo effects of mucosally administered inositol phospholipids

Preparation of the composition for mucosal administration of PI: 2 mg bovine liver PI (Avanti Polar Lipids; catalog number 830042) was dried under a stream of nitrogen and suspended in 1 ml of physiological saline (0.9 % NaCl) by pipetting up and down several times. This 2 mg/ml stock solution was diluted 10-fold in physiological saline to obtain a 0.2 mg/ml suspension.

### Example 2A : Animal Model for Colitis

On day 0 male wild type black/6 mice (Charles Rivers) of 7-10 weeks old were sensitized epicutaneously with 150 µl 3% 4-ethoxymeyhylene-2-phenyl-oxazolin-5-one (oxazolone; Sigma) in 100% ethanol. At day 5, mice were challenged, under anesthesia of ketamine (80 mg/kg) and xylazine (10 mg/kg), by rectal administration of 100 µl 2% oxazolone in aqueous ethanol (50%). Twenty minutes prior to challenge mice were treated intra-rectally with 100 µl saline (control) or PI ( 2 mg/ml). All three groups contained 8 mice. Mice were weighted at day 5, 6, 7, and 8 and were sacrificed at day 8.

Colonic tissue was fixed with 4% natural buffered formalin solution, embedded in paraffin, cut into 5 µ tissue sections, and stained with hematoxylin and eosin (H&E). Using a score that has been described previously for oxazolone colitis (REF), the stained sections were examined for evidence of colitis. Spleen, axillary lymph nodes (ALN),mesenteric lymph nodes (MLN) and iliacal lymph nodes (ILN) were isolated and stimulated ex-vivo with either PBS (control) or with aCD3/aCD28 (100 µl of 2 µg/ml each) in IMDM medium. After 48 and 72 hours, the production of IL-2 was measured by means of ELISA (Beckton & Dickinson).

### Results:

Local treatment with PI through colonic administration prior to the induction of a hapten-induced colitis in mice resulted in diminished weight loss and increased survival. As shown in Fig. 5, intrarectal treatment of mice with IPL suppressed IL-2 production upon ex-vivo stimulation of intestinal T cells. These data indicate that IPL can be used as prophylaxis or treatment of human Inflammatory Bowel Diseases, such as ulcerative colitis.

### Example 2B: Animal Model for Asthma

On days one and eight male wildtype Balb/c mice (Charles Rivers) of 7-10 weeks old were sensitized by intra-peritoneal (i.p.) injection of 20 µg of OVA (Sigma), emulsified in 2.25 mg of alum (AlumImuject; Pierce). On days 15, 16 and 17 mice were sedated and received 80 µl of one of three solutions of PI (0 µg/ml, 200 µg/ml or 2 mg/ml) intra-tracheally (i.t). Subsequently the mice were challenged by 20 minutes of inhalation exposure to 60 µl of aerosols containing OVA (1% in saline). All three groups contained 8 mice. Mice were sacrificed at day 18.

Lungs were flushed with 3 ml saline and the cells in this Broncho Alveolar Lavage (BAL) were identified and quantified through FACS analysis. Subsequently the lungs were freeze-fixed, cut into 5 µm tissue sections and stained with May-Grunwald-Giemsa (MGG). The stained sections were examined for the presence of inflammatory cell infiltration such as eosinophils.

### Results:

This model for airway hyper reactivity shows that intra-tracheal treatment with PI strongly inhibits the typical influx of eosinophils that is associated with asthma. Analysis of the lungs confirmed that PI reduces the histopathological changes that are characteristic for this model. These data indicate that IPL can be suitably used for the prophylaxis or treatment of human asthma.

## Claims

1. A pharmaceutical composition adapted for topical administration comprising as an active ingredient a diacylglycerol phosphatidyl inositol (PI) and a pharmaceutically acceptable carrier or diluent.

2. A pharmaceutical composition according to claim 1, wherein the ratio of saturated versus unsaturated fatty acids present in said PI is at least 1.0, preferably at least 1.1.

3. A pharmaceutical composition according to any of the preceding claims, wherein said PI is of natural origin, preferably derived from mammalian tissue, more preferably liver, most preferably bovine liver.

4. A pharmaceutical composition according to any of the preceding claims, wherein said PI makes up less than 50%, preferably less than 30%, more preferably less than 25% of the dry weight of the composition.

5. A pharmaceutical composition according to any of the preceding claims, wherein the composition is in the form of an ointment, paste, cream or patch.

6. A pharmaceutical composition according to any of the preceding claims, further comprising an other anti-inflammatory drug, preferably aminosalicylate or corticosteroid.

7. A pharmaceutical composition according to any of the preceding claims for use in the treatment of psoriasis.

8. Diacylglycerol phosphatidyl inositol (PI) for use in the treatment of psoriasis.

9. Diacylglycerol phosphatidyl inositol (PI), wherein said PI is of natural origin, preferably derived from mammalian tissue, more preferably liver, most preferably bovine liver.

10. Method for preparing a pharmaceutical composition, which composition comprises as active ingredient diacylglycerol phosphatidyl inositol (PI), comprising preparing a non-liposomal suspension of said active ingredient in an aqueous physiological acceptable carrier.

11. Method according to claim 10, wherein said pharmaceutical composition is formulated as ointment, paste, cream or patch.
